# EUROPEAN PATENT APPLICATION

(11) **EP 1 435 606 A1**
(43) Date of publication of application: **07.07.2004**
(21) Application number: 03250060.5
(22) Date of filing: 03.01.2003
(51) Int. Cl.: G10L 17/00

(54) **Electronic baby-soothing device**

(71) Applicant: Hung, Hung Wen, Hsin Tien, Taipei Hsien (TW)
(72) Inventor: Hung, Hung Wen, Hsin Tien, Taipei Hsien (TW)
(74) Representative: Smith, Norman Ian

(57) **Abstract**

An electronic baby soothing device with wireless remote monitor, which is capable of being automatically activated by baby's crying sound, to responsively generate user prerecorded voice of the baby's mother/father/nanny, manufacturer/user prerecorded soothing sounds and flashing-light effects to soothe the crying baby, and wireless transmitting sound in the vicinity of baby to a portable same wireless frequency's wireless remote receiving device, which is capable of being automatically receiving the sounds in the vicinity of baby and generate vibration, flashing-light alert effects, to alert the baby's caretaker the condition of baby being crying, allowing the baby's caretaker wireless remotely to monitor the sleeping baby anytime..

## Description

### BACKGROUND OF THE INVENTION

### 1. Field of the Invention:

This invention relates to electronics technology, and more particularly to an electronic baby-soothing device with wireless remote monitor, in which a baby-side unit is capable of being automatically activated by the crying sound of baby, to responsively generate user prerecorded voice of parents/nanny, and/or factory-built soothing sounds, and/or light effects for soothing crying baby, and converts/transmits sounds in the vicinity of the baby to wireless signals, and a caretaker-side unit is able to receives/converts the wireless signals to audio sounds, and/or vibration and/or light effects for alerting the baby's caretaker.

### 2. Description of Related Art:

Taking care of babies is a stressful experience for all parents/nanny, particularly in the event that a baby cries just after waking up, and the baby's parents happens to be busily engaged in some other matters, such as away for a short while working in garden, garage, laundry room, or taking a dump, or taking a shower. If a parent hears the continuous crying sound from the awake baby in these situations, it is undoubtedly that the parent will be under high stress, hastily pause what they are working on and rush to the baby's place to check the condition of crying baby, and soothe the awake crying baby into calm state. In the long run, such stresses would often cause some mental or physiological disorders to the baby's parents. Worst of all, if a parent doesn't hear the awake baby's crying sound while taking a shower or taking a dump, it would be undoubted that the baby would continuously cry for a period of time, before the baby's caretaker notices this condition.

Presently, there are various kinds of electronic baby-soothing devices in the market, they can produce soothing sound and/or flashing-light effect, such as lullabies, songs, and music. But these conventional devices, however, has some drawbacks; (1) they require manual operations from the baby's caretaker in order to activate these baby-soothing functions, cannot automatically activated by the crying sound of baby, and so on, they cannot immediately generate soothing sound and/or flashing-light to soothe the crying baby before the baby's caretaker from other places comes to the crying baby; (2) they are not able to allowing the consumer to record preferred sounds, such as voice of the baby's mother/father, and so on, they cannot automatically playback the baby's familiar voice of mother/father while the baby is crying, and to soothe it before the baby's parents rush back from other places, the baby's familiar voice of mother/father can let awake crying baby (especially, known sounds/unknown human stage baby) fell warm, save, and not alone. and cannot be substituted by lullabies, song, music or various other kinds of amusing sounds; and (3) these conventional devices lacks wireless capability to transmit sounds in the vicinity of baby to the baby's caretaker, so the baby's caretaker is unable to wireless remotely monitor the sleeping baby.

There are wireless sound transmitter/receiver devices in the market, they can wireless transmitting/receiving crying sound of baby. But one drawback to these conventional devices, however, is that they lacks baby-soothing functions, and so on , they cannot automatically activated by the baby's crying sound, to responsively generate user prerecorded voice of the baby's mother/father, manufacturer/user prerecorded lullabies, songs, music and/or flashing-light effects to soothe the crying baby into calm state, before the baby's parents comes to the crying baby. In the event that a baby cries just after waking up, and the baby's mother/father happens to be busily engaged in some other matters, such as away for a short while working in garden, garage, laundry room and with dirty hands, or taking a dump, or taking a shower. it would be undoubted that the baby's mother/father need to take a period of time and after clean hands, and rush back to soothe the awake crying baby, and during this time the continuous crying sound from an awake baby, would nerve-racking the baby's mother/father under stressful condition, before she/he comes to check the baby's condition. In the long run, such pressure is not good to the parents' mental health, so these conventional wireless transmitter/receiver devices, still not very good to the baby and the baby's parents.

### SUMMARY OF THE INVENTION

It is therefore an objective of this invention to provide an electronic baby-soothing device, which is capable of being automatically activated by the crying sound of baby, to responsively generate user prerecorded voice of the baby's mother/father/nanny, manufacturer/user prerecorded lullabies, songs, music, to soothe the crying baby into calm state, and/or flashing-light effects to attract the baby attention from crying, before the baby's caretaker from other places comes to the crying baby, and the various kinds of soothing sounds not only factory-built by the manufacturer, but also allowing the consumer to record preferred sounds by themselves'.

It is another objective of this invention to provide an electronic baby-soothing device with wireless remote monitor, which comprises one baby-side unit and one caretaker-side unit. Wherein the baby-side unit is capable of being automatically activated by the crying sound of baby, to responsively generate user prerecorded voice of the baby's mother/father/nanny, manufacturer/user prerecorded lullabies, songs, music, to soothe the crying baby into calm state, and/or flashing-light effects to attract the baby attention from crying, before the baby's caretaker from other places come to the crying baby, and the various kinds of soothing sounds not only factory-built by the manufacturer, but also allowing the consumer to record preferred sounds by themselves', and wireless transmitting the sound of crying baby and/or sounds in the vicinity of the baby. And wherein the caretaker-side unit is capable of being automatically wireless receiving/reproducing the baby's crying sound and/or sounds in the vicinity of baby, and/or flashing-light alert effect, and/or vibration alert effect, as to alert the baby's caretaker the condition of the baby being crying. So the invention allows the baby's caretaker to comes to the crying baby with ease, and when the baby's caretaker is situated at a distant place from the baby, still can wireless remotely monitor the sleeping baby anytime.

An electronic baby-soothing device of this invention's first objective, which is characterized by that is capable of being automatically activated by baby's crying sound, to responsively generate voice of the baby's mother/father/nanny, soothing sound and/or flashing-light effects to soothe the crying baby into calm state, before the baby's caretaker from other places come to the crying baby. and the soothing sounds not only factory-built by the manufacturer, but also allowing the consumer to record preferred sounds by themselves'.

An electronic baby-soothing device with wireless remote monitor of this invention's second objective, which is characterized by that the baby-side unit's baby-soothe device is further capable of being, automatically converter the baby's crying sound and/or sounds in the vicinity of the baby to wireless signals and transmit it to the ether; and the caretaker-side unit is a portable same wireless frequency's remote receiving device, and be carried by the baby's caretaker, which is capable of being automatically receives the wireless signals and converter it to audio sound, and/or automatically activated by the wireless signals, to responsively generate vibration, flashing-light alert effects, to alert the baby's caretaker the condition of the baby being crying.

The invention has the following advantage over prior art, (1) the invention allows the crying baby to be soothed automatically by machine-generated voices of the baby's mother/father/nanny, lullabies, songs, music, and/or magic flashing-light attract baby attention from crying, before the baby's caretaker comes to the crying baby, when the baby's caretaker happens to be busily engaged in some other matters or away for a short; (2) the invention is able to playback the consumer prerecorded voice of mother/father, the baby's familiar voice of mother/father can let awake crying baby (especially, known sounds/unknown human stage baby) fell warm, save, and not alone, and cannot be substituted by lullabies, songs, music or various other kinds of amusing sounds; (3) the invention allows the baby's caretaker temporary leave from the sleeping baby to other places, to process some homework, private matters, or take a break, still can wireless remotely monitor the baby sleeping/crying anytime. Therefore, the invention not only good to the mental health of baby, but also can reduce the mental pressure of baby's caretaker , and allows the baby's caretaker to comes to the awake crying baby with ease, and when the baby's caretaker is situated at a distant place from the baby, still can wireless remotely monitor the sleeping baby anytime. The invention is therefore more advantageous to use than the prior art.

### BRIEF DESCRIPTION OF DRAWINGS

The invention an electronic baby-soothing device with wireless remote monitor, can be more fully understood by reading the following detailed description of the preferred embodiments, with reference made to the accompanying drawings, wherein:
FIG. 1 is a schematic diagram showing the conceptual model of an electronic baby-soothing device, according to a first preferred embodiment of the invention; and
FIG. 2 is a schematic diagram showing the conceptual model of an electronic baby-soothing device with wireless remote monitor, according to a second preferred embodiment of the invention.

### DETAILED DESCRIPTION OF PREFERRED EMBODIMENTS

An electronic baby-soothing device with wireless remote monitor, according to the invention is disclosed by way of preferred embodiments in the following with reference to the accompanying drawings.

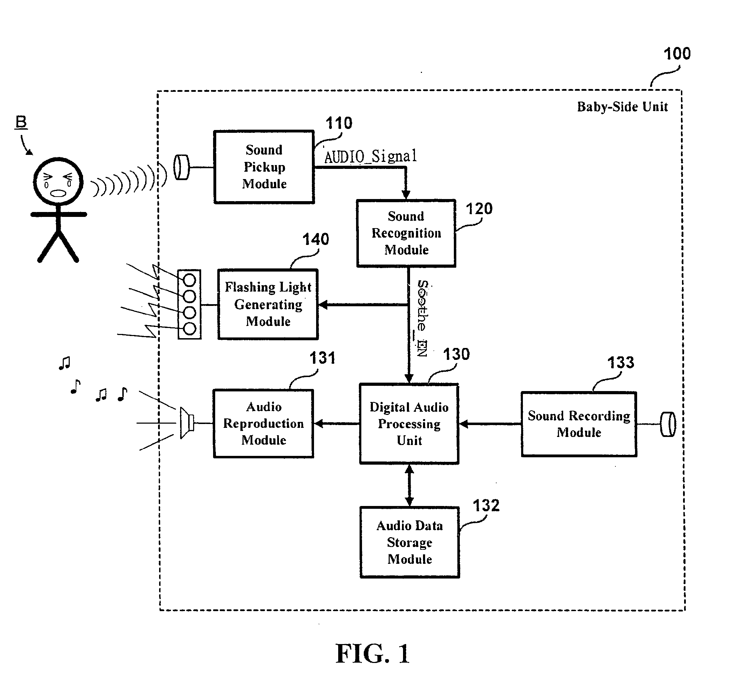

### First Preferred Embodiment (FIG. 1)

FIG. 1 is a schematic diagram showing the conceptual model of the electronic baby-soothing device according to a first preferred embodiment of the invention. This embodiment of the invention includes only a baby-side unit **100**.

As shown, in use, the baby-side unit **100** is put beside a baby **B** (or two or more babies), and which is capable of being automatically activated by the crying sound of the baby **B** to responsively generate various kinds of sound and/or flashing light effects, such as prerecorded voice of parents/nanny, lullabies, songs, music, or various other kinds of amusing sounds, for the purpose of soothing the crying baby **B** into calm state before the baby's caretaker from other places comes to the crying baby **B**.

The baby-side unit **100** comprises: (a) a sound pickup module **110**; (b) a sound recognition module **120**; and (c) means for generating at least one soothing effect, which includes a digital audio processing unit **130**, an audio reproduction module **131**, an audio data storage module **132**, a sound recording module **133**, and a flashing-light generating module **140**.

The sound pickup module **110** is capable of picking up natural sounds from the surrounding environment (which might include the crying sound of the baby **B** or any background noise), and processing the picked sound into an electronic-form audio signal *AUDIO_SIGNAL.* Since the functionality of this sound pickup module **110** is well known in the art of electronics, detailed description of the internal circuit structure thereof will not be given here in this specification.

The sound recognition module **120** is capable of recognizing whether the output audio signal *AUDIO_SIGNAL* from the sound pickup module **110** is a baby's crying sound or a background noise to thereby generate a soothe-enable signal *SOOTHE_ENABLE* if the audio signal *AUDIO_SIGNAL* is indicative of a baby's crying sound. The functionality of this sound recognition module **120** can be implemented by various methods, including, for example, a frequency-pickup method or a voice-recognition method.

The frequency-pickup method utilizes a filter having a bandpass bandwidth matched to the frequency of baby's crying sound, i.e., if the audio signal *AUDIO_SIGNAL* is baby's crying sound, it will pass this filter. Therefore, when the filter has a soothe-enable signal *SOOTHE_ENABLE* output, it indicates that the audio signal is indicative of a baby's crying sound.

The voice recognition method utilizes advanced digital voice recognition technology to recognize, the audio signal *AUDIO_SIGNAL* be converted to digital form and be recognized by manufacture prerecorded program, if indicative of a baby's crying sound, then a soothe-enable signal *SOOTHE_ENABLE* output. Since this voice recognition method is a well-known technology in the art of electronics, detailed description of the internal circuit structure thereof will not be given here in this specification.

The soothe-enable signal *SOOTHE_ENABLE* is used to trigger the operations of the digital audio processing unit **130** and the flashing-light generating module **140**.

In response to the soothe-enable signal *SOOTHE_ENABLE,* the digital audio processing unit **130** retrieves one or more audio clips from the audio data storage module **132** and transfers these audio clips to the audio reproduction module **131** for reproduction by the audio reproduction module **131** into natural sounds. These pieces of soothing sound include, for example, voice of parents/nanny, lullabies, songs, music, or various other kinds of amusing sounds that have pacify effect, and these pieces of soothing sound can be factory-built by the manufacturer into the audio data storage module **132** or recorded by the consumer by means of the sound recording module **133** into the audio data storage module **132**. Since the functionality of the audio reproduction module **130** is well known in the art of electronics, detailed description of the internal circuit structure thereof will not be given here in this specification.

The audio data storage module **132** can be for example a memory module or a disk storage module, which is capable of storing digitized audio data of any kinds of sounds. Preferably, the audio data storage module **132** includes a read-only memory module (not shown) for storing factory-built pieces of audio data, and a rewrittable memory module (not shown), such as flash memory, for storing the consumer (i.e., parents, nanny) preferred pieces of audio data that are recorded by means of the sound recording module **133**.

The flashing-light generating module **140** is capable of generating a predesigned pattern of flashing lights in the event of the presence of the soothe-enable signal *SOOTHE_ENABLE* from the sound recognition module **120.** This flashing light effect can be produced by, for example, an array of LEDs (light-emitting diodes) which is programmed to emit an array of light beams according to a determined order and manner.

In use, the baby-side unit **100** is put beside the baby **B**. In the event that the baby **B** cries, the crying sound from the baby **B** will be picked by the sound pickup module **110** and processed by the same into an electronic-form audio signal *AUDIO_SIGNAL* and then transfers this audio signal *AUDIO_SIGNAL* to the sound recognition module **120** which is capable of recognizing whether the audio signal *AUDIO_SIGNAL* is indicative of a baby's crying sound, and if YES, generating a soothe-enable signal *SOOTHE_ENABLE* to enable the operations of both the audio reproduction module **130** and the flashing-light generating module **140.** In response to the soothe-enable signal *SOOTHE_ENABLE,* the audio reproduction module **130** reproduces one or more prerecorded pieces of soothing sound that are stored in the audio data storage module **132,** such as voice of parents/nanny, lullabies, songs, music, or various other kinds of sounds that have pacify effect; and meanwhile the flashing-light generating module **140** generates a predesigned pattern of flashing lights to provide an amusing light effect to attract the baby **B** attention from crying. By the combination of the soothing sounds from the audio reproduction module **130** and the amusing flashing-light effect from the flashing-light generating module **140**, it can help soothe the crying baby **B** into calm state.

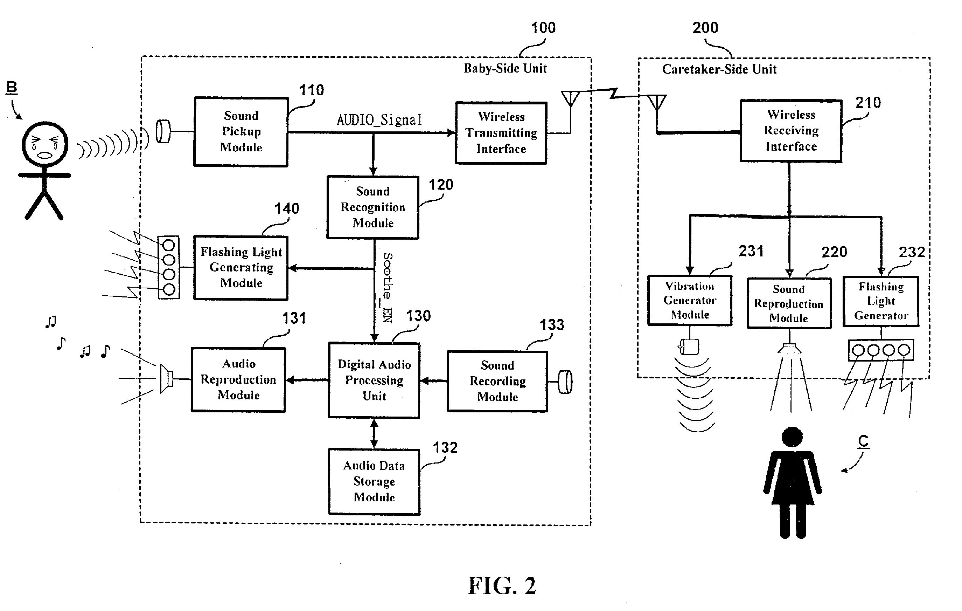

### Second Preferred Embodiment (FIG. 2)

FIG. 2 is a schematic diagram showing the conceptual model of an electronic baby-soothing device with wireless remote monitor, according to a second preferred embodiment of the invention. The second embodiment differs from the first one particularly is that the second embodiment further comprises a caretaker-side unit **200** used in conjunction with the baby-side unit **100**, and baby-side unit **100** further comprises a wireless transmitting interface **150**.

The caretaker-side unit **200** is a portable wireless electronic device carried by the baby's caretaker **C**, i.e., parent or nanny. In the event of the baby **B** crying and/or sounds in the vicinity of baby **B**, the baby-side unit **100** will modulating the sounds to wireless signals and transmitting to the ether, and the same wireless frequency's caretaker-side unit **200** automatically receiving the wireless signals from the ether, and demodulating the wireless signals to audio signals, and the audio signals via sound reproduction module process and reproduce the baby **B** crying sounds and/or sounds in the vicinity of baby **B**, and automatically activated by the audio signals to responsively generate a human-perceivable vibrational alert effect, and generate a human-visible flashing light alert effect, to alert the baby's caretaker **C** the condition of the baby **B** being crying, and/or sounds in the vicinity of baby **B**.

To implement this invention second preferred embodiment, on the baby side, the baby-side unit **100**, in addition to the sound pickup module **110**, the sound recognition module **120**, the audio reproduction module **130**, the audio data storage module **132**, the sound recording module **133**, and the flashing-light generating module **140**, and further comprises a wireless transmitting interface **150** coupled to the sound pickup module **110**, and on the caretaker side, the caretaker-side unit **200** comprises a wireless receiving interface **210**, a sound reproduction module **220**, and a vibration generator **231** and a flashing-light generator **232**.

In this invention second preferred embodiment, the sound pickup module **110**, the sound recognition module **120**, the audio reproduction module **130**, the audio data storage module **132**, the sound recording module **133**, and the flashing-light generating module **140** all operate in the same manner as the first embodiment shown in FIG. 1, so description thereof will not be repeated herein.

When the sound pickup module **110** picks an audio signal *AUDIO_SIGNAL* from sounds in the vicinity of baby **B**, it is directly transferred via the wireless transmitting interface **150** modulating the audio signals *AUDIO_SIGNAL* to wireless signals and transmitting to the ether. On the caretaker side, the same frequency wireless receiving interface **210** in the caretaker-side unit **200** is capable of automatically receiving the wireless signals from the ether and demodulating the wireless signals to audio signals, the demodulated audio signals via sound reproduction module **220** process and reproduce audio sounds, allowing the baby's caretaker **C** to remote monitoring the condition of the baby **B** by sounds; i.e., if the baby **B** cries or sounds in the vicinity of baby **B**, it will be promptly heard by the baby's caretaker **C** from the sound reproduction module **220**.

In addition, a vibration generator **221** is used to generate a human-perceivable vibrational signal to alert the holder of the caretaker-side unit **200;** and moreover, a flashing-light generator **223** is used to generate a human-visible flashing light signal to alert the holder of the caretaker-side unit **200**. Since the sound alert signal may fail to be heard by the baby's caretaker **C** in a noisy environment, or the flashing light alert signal may fail to be seen by the baby's caretaker **C** if the baby's caretaker **C** is attentive on something else, or the vibration alert signal may fail to be sensed by the baby's caretaker **C**, but the redundant provision of the vibration generator **231** and the flashing-light generator **232** in addition to the sound reproduction module **220** would allow the baby's caretaker **C** to better wireless remote monitor the condition of the baby **B**.

When the baby's caretaker **C** perceives the alerting signal from the caretaker-side unit **200** but she/he is currently busily engaged, such as taking a dump or taking a shower in the bathroom, cooking in the kitchen, or speaking important matters on the phone, or away for a short while working in garden, garage or laundry room, the baby's caretaker **C** can nevertheless take ease or proceed on the current doings and come to the baby at a later time since the baby-side unit **100** can automatically generate soothing sounds and flashing light effect to soothe the crying baby **B** into calm state before she/he comes to the crying baby **B** as described previously with reference to FIG. 1.

In practical implementation, the baby-side unit **100** can be installed, for example, inside a toy such as a teddy bear, so that when the baby **B** cries, the teddy bear can automatically produce the soothing sound from its mouth, and flashing-light effect from its eyes or other body parts, and caretaker-side unit **200** can be installed, for example, inside a geometrical shape body. And such application of the electronic baby-soothing device with wireless remote monitor of the invention, the baby-side unit in toy and the caretaker-side unit in geometrical shape body are an arbitrary design choices on the manufacturer side, which are non-essential to the spirit of the invention, so detailed description about this matter will not be given here in this specification.

### Conclusion

In conclusion, an electronic baby-soothing device of this invention's first objective, which is characterized by that is capable of being automatically activated by baby's crying sound, to responsively generate voice of the baby's mother/father/nanny, soothing sound and/or flashing-light effects to soothe the crying baby into calm state, before the baby's caretaker from other places come to the crying baby. and the soothing sounds not only factory-built by the manufacturer, but also allowing the consumer to record preferred sounds by themselves'.

An electronic baby-soothing device with wireless remote monitor of this invention's second objective, which is characterized by that the baby-side unit's baby-soothe device is further capable of being , automatically converter the baby's crying sound and/or sounds in the vicinity of the baby to wireless signals and transmit it to the ether; and the caretaker-side unit is a portable same wireless frequency's remote receiving device, and be carried by the baby's caretaker, which is capable of being automatically receives the wireless signals and converter it to audio sound, and/or activated by the wireless signals, to responsively generate vibration, flashing-light alert effects, to alert the baby's caretaker the condition of the baby being crying. So the invention allows the baby's caretaker to comes to the crying baby with ease, and when the baby's caretaker is situated at a distant place from the baby, still can wireless remotely monitor the sleeping baby anytime.

The invention has the following advantage over prior art, (1) the invention allows the crying baby to be soothed automatically by machine-generated voices of the baby's mother/father/nanny, lullabies, songs, music, and/or magic flashing-light attract baby attention from crying, before the baby's caretaker comes to the crying baby, when the baby's caretaker happens to be busily engaged in some other matters or away for a short; (2) the invention is able to playback the consumer prerecorded voice of mother/father, the baby's familiar voice of mother/father can let awake crying baby (especially, known sounds/unknown human stage baby) fell warm, save, and not alone, and cannot be substituted by lullabies, songs, music or various other kinds of amusing sounds; (3) the invention allows the baby's caretaker temporary leave from the sleeping baby to other places, to process some homework, private matters, or take a break, still can wireless remotely monitor the baby sleeping/crying anytime. Therefore, the invention not only good to the mental health of baby, but also can reduce the mental pressure of baby's caretaker , and allows the baby's caretaker to comes to the awake crying baby with ease, and when the baby's caretaker is situated at a distant place from the baby, still can wireless remotely monitor the sleeping baby anytime. The invention is therefore more advantageous to use than the prior art.

The invention has been described using exemplary preferred embodiments. However, it is to be understood that the scope of the invention is not limited to the disclosed embodiments. On the contrary, it is intended to cover various modifications and similar arrangements. The scope of the claims, therefore, should be accorded the broadest interpretation so as to encompass all such modifications and similar arrangements.

## Claims

1. An electronic baby-soothing device with wireless remote monitor, which comprises:
a sound pickup module, which is capable of picking up crying sound from a baby and/or sound in the vicinity of baby and processed the picked sound into an electronic-form audio signal; and
a sound-recognition module, which is capable of recognizing whether the output audio signal from the sound pickup module is indicative of baby's crying sound to thereby generate a soothe-enable signal if the audio signal is indicative of baby's crying sound; and
means for generating at least one soothing effect in response to the soothe-enable signal from the sound-recognition module.

2. The electronic baby-soothing device with wireless remote monitor of claim 1, wherein the soothing effect generating means includes:
an audio data storage module for storing at least one piece of prerecorded soothing sound which is factory-built into the audio storage module by manufacturer; and
an audio reproduction module for reproducing the prerecorded piece of soothing sound stored in the audio data storage module in response to the soothe-enable signal from the sound-recognition module.

3. The electronic baby-soothing device with wireless remote monitor of claim 2, wherein the soothing effect generating means further includes:
a sound-recording module, which allows user to record a piece of user-preferred sound into the audio data storage module for reproduction by the audio reproduction module in response to the soothe-enable signal from the sound recognition module.

4. The electronic baby-soothing device with wireless remote monitor of claim 2, wherein the soothing effect generating means includes:
a flashing-light generating module, which is capable of generating a flashing-light effect in response to the soothe-enable signal from the sound-recognition module.

5. The electronic baby-soothing device with wireless remote monitor of claim I, further comprising:
a wireless transmitting interface, installed on the baby-side unit which is capable of modulating the sound of crying baby and/or sound in the vicinity of baby to wireless form signal and transmitting to the ether; and
a caretaker-side unit, which includes:
a wireless receiving interface, which is same wireless frequency with baby-side unit's wireless transmitting interface and which is capable of receiving the wireless form signal from the ether and the wireless form signal from baby-side unit's wireless transmitting interface and demodulating the wireless form signal to electronic-form audio signal; and
an alerting-signal generating module, which is coupled to the wireless receiving interface, and which is capable of generating at least one alerting signal in human-perceivable form to alert holder of the caretaker-side unit of the condition of the baby being crying or sound in the vicinity of baby.

6. The electronic baby-soothing device with wireless remote monitor of claim 5, wherein the alerting-signal generating module includes:
a sound reproduction module, which is coupled to the wireless receiving interface and which is capable of reproducing sound in the vicinity of baby to thereby alert holder of the caretaker-side unit of the condition of the baby being crying or sound in the vicinity of baby.

7. The electronic baby-soothing device with wireless remote monitor of claim 5, wherein the alerting-signal generating module includes:
a vibration generator, which is coupled to the wireless receiving interface and which is capable of generating a human-perceivable vibrational signal to alert holder of the caretaker-side unit of the condition of the baby being crying or sound in the vicinity of baby.

8. The electronic baby-soothing device with wireless remote monitor of claim 5, wherein the alerting-signal generating module includes:
a flashing-light generator, which is coupled to the wireless receiving interface and which is capable of generating a human-visible flashing light signal to alert holder of the caretaker-side unit of the condition of the baby being crying or sound in the vicinity of baby.

9. An electronic baby-soothing device with wireless remote monitor, which comprises:
(a) a baby-side unit; and
(b) a caretaker-side unit;
wherein the baby-side unit includes:
a sound pickup module, which is capable of picking up crying sound from a baby and/or sound in the vicinity of baby and processed the picked sound into an electronic-form audio signal; and
a sound-recognition module, which is capable of recognizing whether the output audio signal from the sound pickup module is indicative of baby's crying sound to thereby generate a soothe-enable signal if the audio signal is indicative of baby's crying sound; and
means for generating at least one soothing effect in response to the soothe-enable signal from the sound-recognition module; and
a wireless transmitting interface, which is capable of modulating the sound of crying baby and/or sound in the vicinity of baby to wireless form signal and transmitting to the ether;
and wherein the caretaker-side unit includes:
a wireless receiving interface, which is same wireless frequency with baby-side unit's wireless transmitting interface and which is capable of receiving the wireless form signal from the ether and the wireless form signal from baby-side unit's wireless transmitting interface and demodulating the wireless form signal to electronic-form audio signal; and
an alerting-signal generating module, which is coupled to the wireless receiving interface, and which is capable of generating at least one alerting signal in human-perceivable form to alert holder of the caretaker-side unit of the condition of the baby being crying or sound in the vicinity of baby.

10. The electronic baby-soothing device with wireless remote monitor of claim 9, wherein the soothing effect generating means includes:
an audio data storage module for storing at least one piece of prerecorded soothing sound which is factory-built into the audio storage module by manufacturer; and
an audio reproduction module for reproducing the prerecorded piece of soothing sound stored in the audio data storage module in response to the soothe-enable signal from the sound-recognition module.

11. The electronic baby-soothing device with wireless remote monitor of claim 10, wherein the soothing effect generating means further includes:
a sound-recording module, which allows user to record a piece of user-preferred sound into the audio data storage module for reproduction by the audio reproduction module in response to the soothe-enable signal from the sound recognition module.

12. The electronic baby-soothing device with wireless remote monitor of claim 10, wherein the soothing effect generating means includes:
a flashing-light generating module, which is capable of generating a flashing-light effect in response to the soothe-enable signal from the sound-recognition module.

13. The electronic baby-soothing device with wireless remote monitor of claim 9, wherein the alerting-signal generating module includes:
a sound reproduction module, which is coupled to the wireless receiving interface and which is capable of reproducing sound in the vicinity of baby to thereby alert holder of the caretaker-side unit of the condition of the baby being crying or sound in the vicinity of baby.

14. The electronic baby-soothing device with wireless remote monitor of claim 9, wherein the alerting-signal generating module includes:
a vibration generator, which is coupled to the wireless receiving interface and which is capable of generating a human-perceivable vibrational signal to alert holder of the caretaker-side unit of the condition of the baby being crying or sound in the vicinity of baby.

15. The electronic baby-soothing device with wireless remote monitor of claim 9, wherein the alerting-signal generating module includes:
a flashing-light generator, which is coupled to the wireless receiving interface and which is capable of generating a human-visible flashing light signal to alert holder of the caretaker-side unit of the condition of the baby being crying or sound in the vicinity of baby.

16. An electronic baby-soothing device with wireless remote monitor, which comprises:
(a) a baby-side unit; and
(b) a caretaker-side unit;
wherein the baby-side unit includes:
a sound pickup module, which is capable of picking up crying sound from a baby and/or sound in the vicinity of baby and processed the picked sound into an electronic-form audio signal; and
a sound-recognition module, which is capable of recognizing whether the output audio signal from the sound-sensitive unit is indicative of baby's crying sound to thereby generate a soothe-enable signal if the audio signal is indicative of baby's crying sound; and
a means for generating at least one soothing effect in response to the soothe-enable signal from the sound-recognition module.
an audio data storage module for storing at least one piece of prerecorded soothing sound which is factory-built into the audio storage module by manufacturer; and
an audio reproduction module for reproducing a prerecorded piece of soothing sound in response to the soothe-enable signal from the sound-recognition module; and
a flashing-light generating module, which is capable of generating a flashing-light effect in response to the soothe-enable signal from the sound-recognition module. and
a wireless transmitting interface, which is capable of modulating the sound of crying baby and/or sound in the vicinity of baby to wireless form signal and transmitting to the ether;
and wherein the caretaker-side unit includes:
a wireless receiving interface, which is same wireless frequency with baby-side unit's wireless transmitting interface and which is capable of receiving the wireless form signal from the ether and the wireless form signal from baby-side unit's wireless transmitting interface and demodulating the wireless form signal to electronic-form audio signal; and
an alerting-signal generating module, which is coupled to the wireless receiving interface, and which is capable of generating at least one alerting signal in human-perceivable form to alert holder of the caretaker-side unit of the condition of the baby being crying or sound in the vicinity of baby.

17. The electronic baby-soothing device with wireless remote monitor of claim 16, wherein the soothing effect generating means further includes:
a sound-recording module, which allows user to record a piece of user-preferred sound into the audio data storage module for reproduction by the audio reproduction module in response to the soothe-enable signal from the sound recognition module.

18. The electronic baby-soothing device with wireless remote monitor of claim 16, wherein the alerting-signal generating module includes:
a sound reproduction module, which is coupled to the wireless receiving interface and which is capable of reproducing sound in the vicinity of baby to thereby alert holder of the caretaker-side unit of the condition of the baby being crying or sound in the vicinity of baby.

19. The electronic baby-soothing device with wireless remote monitor of claim 16, wherein the alerting-signal generating module includes:
a vibration generator, which is coupled to the wireless receiving interface and which is capable of generating a human-perceivable vibrational signal to alert holder of the caretaker-side unit of the condition of the baby being crying or sound in the vicinity of baby.

20. The electronic baby-soothing device with wireless remote monitor of claim 16, wherein the alerting-signal generating module includes:
a flashing-light generator, which is coupled to the wireless receiving interface and which is capable of generating a human-visible flashing light signal to alert holder of the caretaker-side unit of the condition of the baby being crying or sound in the vicinity of baby.
